# EUROPÄISCHE PATENTSCHRIFT

(11) **EP 1 066 041 B1**
(45) Veröffentlichungstag und Bekanntmachung des Hinweises auf die Patenterteilung: **09.11.2005**
(21) Anmeldenummer: 99907318.2
(22) Anmeldetag: 10.02.1999
(51) Int. Cl.: A61K 31/565, A61K 38/09, A61P 15/08

(54) **VERWENDUNG VON 17-alpha-ESTRADIOL DERIVATEN ZUR THERAPIE VON NEBENWIRKUNGEN WÄHREND UND/ODER NACH EINER GnRHa-THERAPIE**
THE USE OF17-ALPHA-ESTRADIOL DERIVATIVES FOR TREATING SIDE-EFFECTS DURING AND/OR AFTER GnRHa THERAPY
UTILISATION DE DERIVES DE 17-ALPHA-'ESTRADIOL POUR LE TRAITEMENT D'EFFETS SECONDAIRES PENDANT OU APRES UNE THERAPIE GnRHa

(30) Priorität: 03.04.1998 DE 19815060
(43) Veröffentlichungstag der Anmeldung: 10.01.2001
(73) Patentinhaber: Schering AG, 13353 Berlin (DE)
(72) Erfinder: WILDT, Ludwig, D-91974 Herzogenaurach-Hauendorf (DE); NEUWINGER, Joachim, D-91054 Erlangen (DE); LICHT, Peter, D-91088 Bubenreuth (DE); DITTRICH, Ralph, D-91056 Erlangen (DE); OETTEL, Michael, D-07743 Jena (DE); HUMMEL, Wolfgang, D-95339 Neuenmarkt (DE)
(74) Vertreter: Cramer, Eva-Maria
(86) Internationale Anmeldenummer: PCT/DE1999/000355
(87) Internationale Veröffentlichungsnummer: WO 1999/051243

(56) Entgegenhaltungen:
- EP-A- 0 402 950
- WO-A-91/01748
- WO-A-91/14704
- WO-A-94/26207
- WO-A-97/21704
- WENZL ET AL: "Effectiveness and Tolerance of a GnRH Agonist (Goserelin) for the Treatment of Symptomatic Endometriosis" WIEN. KLIN. WOCHENSCHR., Bd. 111, Nr. 7, 1999, Seiten 283-288, XP002113059
- SURREY: "Steroidal and Non-Steroidal "Add-Back" Therapy: Extending Safety and Efficacy of GnRH Agonists in the Gynecologic Patient" FERTIL STERIL, Bd. 64, 1995, Seiten 673-685, XP002113752
- FREUNDL ET AL: "Steroidal Add-Back Therapy in Patients with GnRH Agonists" GYNECOL. OBSTET. INVEST., Bd. 45, Nr. s1, 1998, Seiten 22-30, XP002113060
- ANTTILA ET AL: "GnRH Agonists: Clinical Application in Management of Endometriosis" ASSISTED REPROD. TECHNOL. / ANDROL., Bd. 9, Nr. 1/2, 1997, Seiten 65-74, XP002113061
- SHERWIN ET AL: ""Add-Back" Estrogen Reverses Cognitive Deficits Induced by a GnRH Agonist in Women with Leiomyomata Uteri" J. CLIN. ENDOCRINOL., Bd. 81, Nr. 7, 1996, Seiten 2545-2549, XP002113062
- HEINER ET AL: "Comparison of a GnRH Agonist and a Low Dose Oral Contraceptive Given Alone or Together in the Treatment of Hirsutism" J. CLIN. ENDOCRIN. METAB., Bd. 80, Nr. 12, 1995, Seiten 3412-3418, XP002113063

## Beschreibung

Die Erfindung betrifft die Verwendung von mindestens einem Wirkstoff aus der Gruppe 17α-Estradiol, dessen chemisch modifizierte Derivate, zur Herstellung pharmazeutischer Präparate zur Therapie von Nebenwirkungen, wie Hitzewallungen, während und/oder nach einer Behandlung mit Analoga oder Antagonisten des Gonadotropin- Releasinghormons (GnRHa-Therapie).

Es ist bekannt, daß die Behandlung mit Analoga oder Antagonisten des Gonadotropin-Releasinghormons (GnRHa-Therapie) eine effektive Therapie von Erkrankungen, deren Intensität von der Aktivität der Gonaden beeinflußt wird, darstellt.
Eine derartige Erkrankung ist beispielsweise das Prostatakarzinom beim Mann.

Nach Schindier, AE., Der Frauenarzt 2:211-214, 1995 leiden 8 bis 12 % der Frauen im reproduktionsfähigen Alter an Endometriose.
Leitsymptome der Endometriose sind neben Dysmennorrhoe auch Dyspareunie sowie zyklische und azyklische Unterbauchschmerzen. Hinzu kommen Sterilitäts- bzw. Infertilitätsprobleme.
Seitdem die Endometriose als progrediente estrogenabhängige Erkrankung identifiziert wurde, gibt es Versuche, neben der operativen Sanierung eine möglichst effektive hormonale Therapie zu etablieren. Dazu wurden in erster Linie ein Regime zur Induktion einer Pseudogravidität (Andrews WC and Larsen GD, Am.J.Obst.Gynecol.118(5): 643-651, 1974), Gestagene (Moghissi KS, J.Obstet. Gynecol. 47: 265-267, 1976) und später Danazol (Schweppe KW, Endometriose 5: 4-11) eingesetzt. Heute stehen zur Induktion eines reversiblen medikamentös induzierten Hypo-Estrogenismus GnRH-Analoga zur Verfügung (Regidor PA et al., Zentralbl.Gynäkol. 118: 283-290, 1996).
Die zur Anwendung gelangenden GnRH-Analoga, wie Goserelin oder Leuprorelin, bremsen die Gonadotropinbildung in der Hirnanhangdrüse, legen so die Estrogenproduktion in den Eierstöcken still und führen u.a. zum Ausbleiben der Regelblutung. Sie müssen entweder einmal monatlich gespritzt oder zweimal täglich als Nasenspray appliziert werden. Die Nebenwirkungen der GnRH-Analoga sind den in den Wechseljahren auftretenden Beschwerden, wie Hitzewallungen, Schweißausbrüche, Kopfschmerzen und Depressionen, ähnlich.
Hitzewallungen und Schweißausbrüche gehören zu den unangenehmsten Nebenwirkungen einer GnRHa-Therapie, die auch beim Mann im Falle des fortgeschrittenen und /oder metastasierten Prostatakarzinoms benutzt wird und somit eine breite Anwendung findet.

Die GnRHa-Therapie als hormonbremsende Behandlung stellt einen palliativen Therapieansatz dar. Damit erhält die Lebensqualität des Patienten während der Behandlung einen besonderen, Stellenwert. Nach Kliesch S et al., Dtsch.med.Wschr.122: 940-945, 1997 ist das Auftreten von Hitzewallungen und den damit verbundenen Schweißausbrüchen bei bis zu 80 % der Patientinnen eine häufige und unerwünschte Begleiterscheinung.
Ein vergleichbares Auftreten von Hitzewallungen bei Männern wird zu 58 % nach Orchiektomie und zu 63 % bei der Therapie mit GnRH-Analoga beschrieben (Kaisary AV et al., Brit.J.Urol. 67: 502 - 508, 1991).

Zur Unterdrückung von Hitzewallungen generell sind bis zum gegenwärtigen Zeitpunkt sowohl bei Frauen als auch bei Männern Estrogene Mittel der Wahl.
In der Meno- und Postmenopause haben sie eine breite Anwendung zur Behandlung klimakterischer Beschwerden gefunden.

Die WO 9721704 zeigt neue Verbindungen als GnRH-Antagonisten u.a. in Kombination mit Estrogenen auch zur Behandlung von Hitzewallungen auf.
Ein Einsatz von Estrogenen ist bei einer Therapie mit GnRH-Analoga nicht ratsam, da die therapierte Grundkrankheit eine estrogenabhängige Erkrankung darstellt (Dizerga GS et al., Fertil. Steril.33: 649-653, 1980) und sich das Krankheitsbild bei der Gabe von Estrogenen verschlechtern würde.
Auch beim Mann unter einer GnRHa-Therapie des Prostatakarzinoms muß die klassische Verabreichung von Estrogenen mit Zurückhaltung betrachtet werden.

Die unter einer Estrogentherapie auftretenden unerwünschten Nebenwirkungen zeigen sich u.a. in metabolischen Effekten, die z.B. zu einer Erhöhung der Bindungsglobuline für Sexualsteroide. Schilddrüsenhormone und Kortisol führen. Sie äußern sich in Spannungen der Brustdrüsen, Kopfschmerzen, Flüssigkeitsretention, Übelkeit, Gewichtszunahme und Depression (Jewelewicz R, Fertil.Steril.67: 1-12, 1997).
Ferner sind bei Männern unter einer Estrogentherapie als wichtige Nebenwirkung kardiovaskulare Komplikationen zu erwarten (Haapiainen R et al.,Brit.J.Urol.66: 94-97, 1990; Stege R et al.,Urologie 34: 398-403, 1996). Auch Feminisierungserscheinungen sind möglich.

Der Erfindung liegt die Aufgabe zugrunde, Wirkstoffe zu finden, die die Herstellung neuer pharmazeutischer Präparate mit hoher Wirksamkeit zur Therapie von Nebenwirkungen, wie Hitzewallungen, während und/oder nach einer Behandlung mit Analoga oder Antagonisten des Gonadotropin-Releasinghormons (GnRHa-Therapie) ermöglichen.

Die Aufgabe wird erfindungsgemäß dadurch gelöst, daß mindestens ein Wirkstoff aus der Gruppe 17α-Estradiol, dessen chemisch modifizierte Derivate, zur Herstellung pharmazeutischer Präparate zur Therapie von Nebenwirkungen, wie Hitzewallungen, während und/oder nach einer Behandlung mit Analoga oder Antagonisten des Gonadotropin-Releasinghormons (GnRHa-Therapie) beim Mann verwendet wird.
Vorteilhafte Ausgestaltungen der Erfindung sind die Verbindungen
17α-Estradiol
ent-17α-Estradiol
4-Methyl-estra-1,3,5(10)-trien-1,17α-diol
4-Methyl-estra-1,3,5(10),6-tetraen-1,17α-diol
4-Methyl-estra-1,3,5(10),6,8-pentaen-1,17α-diol
14α,15α-Methylenestra-1,3,5(10),8-tetraen-3,17α-diol oder
14β,15β-Methylenestra-1,3,5(10),8-tetraen-3,17α-diol

Gegenstand der vorliegenden Erfindung sind auch pharmazeutische Präparate zur oralen und parenteralen, incl, topischen, rektalen, subcutanen, intravenösen, intramuskulären, intraperitonealen, intranasalen, intravaginalen, intrabukkalen oder sublingualen Applikation, die neben üblichen Träger- und Verdünnungsmitteln eine im Anspruch 1 oder 2 aufgezeigte Verbindung als Wirkstoff enthalten.

Dabei können es sich um Peroralia, z.B. Tabletten, Kapseln und Dragees oder Lösungen, um perkutane Zubereitungsformen, z.B. Transdermale Therapeutische Systeme (TTS) oder Gele, Sprays oder Salben, um intranasale Zubereitungsformen wie Nasenspray oder Nasentropfen, rektale Zubereitungsformen wie Suppositorien und um Parenteralia, z.B.: Implantate, Presslinge und Ampullen handeln.

Die Zubereitungsformen werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Die Vorteile der Erfindung ergeben sich im wesentlichen dadurch, daß Wirkstoffe zur Herstellung pharmazeutischer Präparate gefunden wurden, welche die herkömmlichen Nebenwirkungen, wie Hitzewailungen, während und/oder nach einer Behandlung mit Analoga oder Antagonisten des Gonadotropin-Releasinghormons (GnRHa-Therapie) beim Mann verhindern.
Gleiches gilt auch für Nebenwirkungen bei anderen hormonsupressiven Therapiemöglichkeiten.

## Patentansprüche

1. Verwendung von mindestens einem Wirkstoff aus der Gruppe 17α-Estradiol oder dessen chemisch modifizierten Derivaten zur Herstellung pharmazeutischer Präparate zur Therapie von Ne benwirkungen, während und/oder nach einer Behandlung mit Analoga oder Antagonisten des Gonadotropin-Releasinghormons beim Mann.

2. Verwendung nach Anspruch 1,
**dadurch gekennzeichnet, daß** die Verbindung
17α-Estradiol,
ent-17α-Estradiol,
4-Methyl-estra-1,3,5(10)-trien-1,17α-diol
4-Methyl-estra-1,3,5(10),6-tetraen-1,17α-diol,
4-Methyl-estra-1,3,5(10),6,8-pentaen-1,17α-diol
14α,15α-Methylenestra-1,3,5(10),8-tetraen-3,17α-diol oder
14β,15β-Methylenestra-1,3,5(10),8-tetraen-3,17α-diol
ist.

## Claims

1. Use of at least one active ingredient from the group of 17α-estradiol or chemically modified derivatives thereof for manufacturing pharmaceutical products for the therapy of side effects during and/or after treatment with analogues or antagonists of gonadotropin-releasing hormone in man.

2. Use according to Claim 1, **characterized in that** the compound is
17α-estradiol
ent-17α-estradiol,
4-methyl-estra-1,3,5(10)-triene-1,17a-diol,
4-methyl-estra,1,3,5(10),6-tetraene-1,17α-diol,
4-methyl-estra-1,3,5(10),6,8-pentaene-1,17α-diol,
14α,15α-methyleneestra-1,3,5(10),8-tetraene-3,17α-diol or
14β,15β-methyleneestra-1,3,5(10),8-tetraene-3,17α-diol.

## Revendications

1. Utilisation d'au moins une substance active du groupe du 17α-estradiol ou de ses dérivés chimiquement modifiés pour la préparation de préparations pharmaceutiques destinées à la thérapie des effets secondaires pendant et/ou après un traitement avec des analogues ou des antagonistes de l'hormone de libération de la gonadotropine chez l'homme.

2. Utilisation selon la revendication 1, **caractérisée en ce que** le composé est
le 17α-estradiol,
l'ent-17α-estradiol,
le 4-méthyl-estra-1,3,5(10)-triène-1,17α-diol
le 4-méthyl-estra-1,3,5(10),6-tétraène-1,17α-diol,
le 4-méthyl-estra-1,3,5(10),6,8-pentaène-1,17α-diol
le 14α,15α-méthylène-estra-1,3,5(10),8-tétraène-3,17a-diol ou
le 14β,15β-méthylène-estra-1,3,5(10),8-tétraène-3,17α-diol.
